# EUROPÄISCHE PATENTANMELDUNG

(11) **EP 2 506 102 A2**
(43) Veröffentlichungstag der Anmeldung: **03.10.2012**
(21) Anmeldenummer: 12154498.5
(22) Anmeldetag: 08.02.2012
(51) Int. Cl.: G05B 19/418, G05B 23/02

(54) **Verfahren zum Verarbeiten eines Saft- und/oder Limonadenprodukts**

(30) Priorität: 01.04.2011 DE 102011006655
(71) Anmelder: Krones AG, 93073 Neutraubling (DE)
(72) Erfinder: Weinzierl, Matthias, 84174 Eching (DE)
(74) Vertreter: Grünecker, Kinkeldey, Stockmair & Schwanhäusser

(57) **Zusammenfassung**

Die Erfindung umfasst ein Verfahren zum Verarbeiten, insbesondere Herstellen und/oder Abfüllen, eines Saft- und/oder Limonadenprodukts in einer Getränkeverarbeitungsanlage, wobei das Saft- und/oder Limonadenprodukt aus einem oder mehreren Ausgangsprodukten erhalten wird, umfassend die Schritte Bestimmen der Farbe und der Trübung wenigstens eines Ausgangsprodukts eines Saft- und/oder Limonadenprodukts und/oder der Farbe und der Trübung des erhaltenen Saft- und/oder Limonadenprodukts mittels wenigstens eines Sensors (6, 7, 8, 9) der Getränkeverarbeitungsanlage, Vergleichen der bestimmten Farbe und Trübung mit einer gespeicherten Farbe und einer gespeicherten Trübung wenigstens eines Ausgangsprodukts eines vorherbestimmten Saft- und/oder Limonadenprodukts und/oder mit einer gespeicherten Farbe und einer gespeicherten Trübung des vorherbestimmten Saft- und/oder Limonadenprodukts, und Betreiben der Getränkeverarbeitungsanlage basierend auf dem Ergebnis dieses Vergleichs.

## Beschreibung

Die vorliegende Erfindung betrifft ein Verfahren zum Verarbeiten, insbesondere Herstellen und/oder Abfüllen, eines Saft- und/oder Limonadenprodukts in einer Getränkeverarbeitungsanlage.

In der Getränkeindustrie werden häufig unterschiedlichste Saft- und/oder Limonadenprodukte hintereinander in derselben Getränkeverarbeitungsanlage verarbeitet. Bei einem Formatwechsel zu einem neuen Produkt kann es vorkommen, dass Reste des zuvor verarbeiteten Saft-und/oder Limonadenprodukts noch teilweise in der Getränkeverarbeitungsanlage vorhanden sind. Dies kann die Qualität des neuen zu verarbeitenden Saft- und/oder Limonadenprodukts negativ beeinflussen.

Auch ist es denkbar, dass nach einem Formatwechsel nicht die korrekten Bestandteile oder Ausgangsprodukte für das neue Saft- und/oder Limonadenprodukt bereitgestellt werden. Beispielsweise kann eine Bedienperson ein falsches Ausgangsprodukt in die Getränkeverarbeitungsanlage einbringen, das dem gewünschten Ausgangsprodukt optisch jedoch ähnlich ist. Wenn dieser Fehler zu spät erkannt wird, kann dies zu kostenintensiven Produktionsausfällen führen.

Daher ist es Aufgabe der vorliegenden Erfindung, ein Verfahren zum Verarbeiten, insbesondere Herstellen und/oder Abfüllen, eines Saft- und/oder Limonadenprodukts in einer Getränkeverarbeitungsanlage bereitzustellen, das ein zuverlässiges Verarbeiten des Saft- und/oder Limonadenprodukts erlaubt.

Die Erfindung stellt ein Verfahren zum Verarbeiten, insbesondere Herstellen und/oder Abfüllen, eines Saft- und/oder Limonadenprodukts in einer Getränkeverarbeitungsanlage bereit, wobei das Saft- und/oder Limonadenprodukt aus einem oder mehreren Ausgangsprodukten erhalten wird, umfassend die Schritte:

Bestimmen der Farbe und der Trübung wenigstens eines Ausgangsprodukts eines Saft-und/oder Limonadenprodukts und/oder der Farbe und der Trübung des erhaltenen Saft-und/oder Limonadenprodukts mittels wenigstens eines Sensors der Getränkeverarbeitungsanlage,

Vergleichen der bestimmten Farbe und Trübung mit einer gespeicherten Farbe und einer gespeicherten Trübung wenigstens eines Ausgangsprodukts eines vorherbestimmten Saft-und/oder Limonadenprodukts und/oder mit einer gespeicherten Farbe und einer gespeicherten Trübung des vorherbestimmten Saft- und/oder Limonadenprodukts, und
Betreiben der Getränkeverarbeitungsanlage basierend auf dem Ergebnis dieses Vergleichs.

Dadurch, dass die Farbe und die Trübung wenigstens eines Ausgangsprodukts eines Saft-und/oder Limonadenprodukts und/oder des fertigen Saft- und/oder Limonadenprodukts gemessen und mit den gespeicherten entsprechenden Größen eines vorherbestimmten oder gewünschten Saft- und/oder Limonadenprodukts verglichen wird, kann ein falsches oder qualitativ nicht ausreichendes Ausgangsprodukt bzw. Abweichungen des erhaltenen Saft- und/oder Limonadenprodukts von gewünschten Spezifikationen erkannt werden. Dadurch kann ein zuverlässigeres Betreiben der Getränkeverarbeitungsanlage erreicht werden. Insbesondere ist dadurch eine Qualitätskontrolle möglich.

Als Verarbeiten eines Saft- und/oder Limonadenprodukts können hierin beliebige Behandlungsschritte verstanden werden, die das Saft- und/oder Limonadenprodukt oder einzelne Ausgangsprodukte davon beinhaltet. Beispielsweise kann das Herstellen und/oder Abfüllen eines Saft- und/oder Limonadenprodukts als Verarbeiten bezeichnet werden.

Als Saftprodukt kann hierin insbesondere ein Getränk verstanden werden, das wenigstens einen auf Pflanzenbasis basierenden Bestandteil umfasst. Insbesondere kann wenigstens ein Ausgangsprodukt des Saftprodukts ein Frucht- und/oder Gemüsesaft sein. Auch ein Sirup kann als Saftprodukt bezeichnet werden.

Als Limonadenprodukt kann ein alkoholfreies Getränk auf Wasserbasis bezeichnet werden, das neben Wasser auch Aromaextrakte, künstliche und/oder natürliche Aromastoffe, Zucker und/oder Süßstoff umfasst. Ein Limonadenprodukt kann auch einen Fruchtsaftanteil und/oder einen Gemüsesaftanteil aufweisen. Der Fruchtsaftanteil und/oder der Gemüsesaftanteil eines Limonadenprodukts kann jedoch geringer sein als der Saftanteil und/oder der Gemüsesaftanteil eines Saftprodukts.

Das Saft- und/oder Limonadenprodukt kann mit Kohlensäure versetzt werden. Dadurch kann ein karbonisierter Softdrink (CSD) erhalten werden.

Als Ausgangsprodukte können hierin Komponenten oder Bestandteile verstanden werden, aus denen das fertige Saft- und/oder Limonadenprodukt, beispielsweise durch Mischen, erhalten wird. Mit anderen Worten können als Ausgangsprodukte Bestandteile verstanden werden, aus denen in der Getränkeverarbeitungsanlage das Saft- und/oder Limonadenprodukt erhalten werden kann. Als Ausgangsprodukte sind insbesondere Wasser, Frucht- und/oder Gemüsesäfte und/oder Milch möglich.

Wenn das Saft- und/oder Limonadenprodukt aus einem, insbesondere genau einem, Ausgangsprodukt erhalten wird, kann das Saft- und/oder Limonadenprodukt auch dem Ausgangsprodukt entsprechen.

Als Trübung kann eine Schwächung der durchgehenden Lichtstrahlung und/oder eine Streuung der Lichtstrahlung aufgrund von in der Flüssigkeit suspendierten Teilchen verstanden werden.

Der Schritt des Vergleichens kann durch ein Steuerungselement der Getränkeverarbeitungsanlage durchgeführt werden. Das Steuerungselement kann beispielsweise eine speicherprogrammierbare Steuerung (SPS) und/oder ein Computer sein. Farbe und Trübung wenigstens eines Ausgangsprodukts eines vorherbestimmten Saft- und/oder Limonadenprodukts und/oder des vorherbestimmten Saft- und/oder Limonadenprodukts können in einem Speicherelement, insbesondere des Steuerungselements, gespeichert sein.

Das Verarbeiten kann ein Herstellen eines Saft- und/oder Limonadenprodukts sein. Mit anderen Worten kann das Verfahren ein Herstellen des Saft- und/oder Limonadenprodukts umfassen. Insbesondere kann das Verfahren ein Herstellen des Saft- und/oder Limonadenprodukts umfassen, wobei das Herstellen ein Mischen von wenigstens zwei Ausgangsprodukten umfasst und wobei das Bestimmen der Farbe und der Trübung vor, während und/oder nach dem Mischen durchgeführt wird.

Wenn das Bestimmen der Farbe und der Trübung vor dem Mischen durchgeführt wird, kann beispielsweise untersucht werden, ob die korrekten Ausgangsprodukte für das gewünschte Saft- und/oder Limonadenprodukt bereitgestellt werden. Wenn das Bestimmen während des Mischens durchgeführt wird, kann das korrekte Mischverhältnis kontrolliert werden. Wenn das Bestimmen nach dem Abfüllen durchgeführt wird, kann beispielsweise kontrolliert werden, ob das hergestellte Saft- und/oder Limonadenprodukt den Spezifikationen eines zugrundeliegenden Rezeptes entspricht.

Das Verarbeiten kann ein Abfüllen eines Saft- und/oder Limonadenprodukts sein. Das Verfahren kann also ein Abfüllen des Saft- und/oder Limonadenprodukts umfassen, wobei das Bestimmen der Farbe und der Trübung vor und/oder während des Abfüllens durchgeführt wird.

Der Schritt des Vergleichens kann insbesondere ein Erkennen des wenigstens einen Ausgangsprodukts des Saft- und/oder Limonadenprodukts umfassen. Mit anderen Worten kann wenigstens ein Ausgangsprodukt eines Saft- und/oder Limonadenprodukts basierend auf der bestimmten Farbe und Trübung, insbesondere eindeutig, erkannt oder identifiziert werden.

Der Schritt des Vergleichens kann auch ein Erkennen des Saft- und/oder Limonadenprodukts, insbesondere basierend auf der bestimmten Farbe und Trübung, umfassen.

Das Betreiben der Getränkeverarbeitungsanlage basierend auf dem Ergebnis des Vergleichs kann ein Anhalten, einen Betriebsmoduswechsel und/oder eine Ausgabe eines Warnsignals umfassen. Wenn beispielsweise ein falsches Ausgangsprodukt erkannt wird, kann die Getränkeverarbeitungsanlage angehalten oder gestoppt und/oder eine Ausgabe eines Warnsignals veranlasst werden. Dies kann beispielsweise durch ein Steuerungselement der Getränkeverarbeitungsanlage geschehen.

Das Warnsignal kann einem optischen und/oder einem akustischem Warnsignal entsprechen. Das Warnsignal kann eine Fehlermeldung umfassen.

Als Betriebsmodus kann eine vorherbestimmte Auswahl oder Einstellung von Betriebsparameterwerten der Getränkeverarbeitungsanlage bezeichnet werden. Mit anderen Worten kann die Getränkeverarbeitungsanlage in einem gewählten Betriebsmodus vorherbestimmte Verarbeitungsschritte in einer vorherbestimmten Weise durchführen. Je nach Betriebsmodus können vorherbestimmte Verarbeitungsschritte auch nicht durchgeführt werden. Beispielsweise kann ein Betriebsmodus einem Standby-Modus, einem Abfüllmodus oder einem Ausleitemodus entsprechen.

Im Standby-Modus können Verarbeitungsfunktionen der Anlage temporär deaktiviert werden, ohne jedoch die Getränkeverarbeitungsanlage komplett abzuschalten. Dadurch können die Verarbeitungsfunktionen bei Bedarf schneller wieder aktiviert werden. Im Abfüllmodus kann das Saft- und/oder Limonadenprodukt verarbeitet werden, insbesondere abgefüllt werden. Im Ausleitemodus kann ein Anfangsprodukt und/oder ein Saft- und/oder Limonadenprodukt aus der Getränkeverarbeitungsanlage ausgeleitet oder ausgeschoben werden, beispielsweise bei einem Formatwechsel auf ein neues zu verarbeitendes Saft- und/oder Limonadenprodukt. Dazu können beispielsweise ein oder mehrere neue Ausgangsprodukte in die Getränkeverarbeitungsanlage eingebracht werden.

Als Betriebsmoduswechsel kann ein Wechsel zwischen zwei unterschiedlichen Betriebsmodi verstanden werden.

Im Ausleitmodus können insbesondere Mischphasen bestimmt werden. Als Mischphasen können Mischungen zwischen einem oder mehreren Ausgangsprodukten und/oder einem Saft-und/oder Limonadenprodukt vor einem Format- oder Produktwechsel und solchen nach dem Format- oder Produktwechsel verstanden werden. Sobald beispielsweise festgestellt wird, dass das vorherig verarbeitete Saft- und/oder Limonadenprodukt im Wesentlichen nicht mehr in der Getränkeverarbeitungsanlage vorhanden ist, kann die Getränkeverarbeitungsanlage beispielsweise in einen Abfüllmodus wechseln.

Die Schritte der oben genannten Verfahren können insbesondere mehrfach, insbesondere periodisch oder nach vorherbestimmten Zeitintervallen, durchgeführt werden. Damit kann eine Onlinequalitätskontrolle der Getränkeverarbeitungsanlage erreicht werden, also eine Kontrolle der Getränkeverarbeitungsanlage während der Produktion. Beispielsweise können die Schritte in einem Intervall von 1 Minute durchgeführt werden. Es kann aber auch ein Intervall von etwa 0,1 bis maximal 5 Sekunden verwendet werden, insbesondere um online die Betriebssicherheit zu gewährleisten. Idealerweise kann das Intervall an die Taktzeit der Steuerung angepasst werden, beispielsweise 8 Messwerte pro Sekunde. So kann eine Veränderung ohne Verlust direkt erfasst werden. Bei sehr großen Volumenströmen kann sogar ein noch kleineres Intervall sinnvoll sein, um Produktverluste zu minimieren.

Das Vergleichen kann insbesondere ein Bestimmen umfassen, ob die bestimmte Farbe und Trübung mit der gespeicherten Farbe und der gespeicherten Trübung gemäß eines vorherbestimmten Kriteriums übereinstimmt. Das vorherbestimmte Kriterium kann vorherbestimmte Toleranzgrenzen spezifizieren. Dadurch kann die gewünschte zu erreichende Qualität oder Zuverlässigkeit flexibel eingestellt werden. Dadurch kann auch Messfehlern bei der Bestimmung der Farbe und der Trübung Rechnung getragen werden.

Das Betreiben der Getränkeverarbeitungsanlage basierend auf dem Ergebnis des Vergleichs kann ein Ändern oder Einstellen wenigstens eines Verarbeitungsparameters der Getränkeverarbeitungsanlage basierend auf dem Ergebnis des Vergleichs umfassen. Beispielsweise kann ein Mischverhältnis beim Mischen mehrerer Ausgangsprodukte basierend auf dem Ergebnis des Vergleichs geändert oder eingestellt werden.

Zusätzlich zu Farbe und Trübung können auch noch eine oder mehrere weitere Eigenschaften des wenigstens einen Ausgangsprodukts und/oder des Saft- und/oder Limonadenprodukts bestimmt und mit der entsprechenden gespeicherten Eigenschaft wenigstens eines Ausgangsprodukts eines vorherbestimmten Saft- und/oder Limonadenprodukts und/oder des wenigstens einen vorherbestimmten Saft- und/oder Limonadenprodukts verglichen werden. Dadurch kann die Zuverlässigkeit des Verfahrens weiter erhöht werden. Beispielhafte weitere Eigenschaften können die elektrische Leitfähigkeit, die Temperatur und/oder die Dichte sein.

Die Erfindung stellt außerdem eine Getränkeverarbeitungsanlage zum Verarbeiten, insbesondere Herstellen und/oder Abfüllen, eines Saft- und/oder Limonadenprodukts bereit, wobei das Saft- und/oder Limonadenprodukt aus einem oder mehreren Ausgangsprodukten erhalten wird, umfassend:
wenigstens einen Sensor zum Bestimmen der Farbe und der Trübung wenigstens eines Ausgangsprodukts eines Saft- und/oder Limonadenprodukts und/oder der Farbe und der Trübung des erhaltenen Saft- und/oder Limonadenprodukts,
ein Speicherelement, in dem die Farbe und die Trübung wenigstens eines Ausgangsprodukts wenigstens eines vorherbestimmten Saft- und/oder Limonadenprodukts und/oder die Farbe und die Trübung des wenigstens einen vorherbestimmten Saft- und/oder Limonadenprodukts gespeichert ist, und
ein Steuerungselement (14), das derart konfiguriert und/oder ausgebildet ist,
dass eine durch den wenigstens einen Sensor bestimmte Farbe und Trübung mit der gespeicherten Farbe und der gespeicherten Trübung wenigstens eines Ausgangsprodukts wenigstens eines vorherbestimmten Saft- und/oder Limonadenprodukts und/oder die Farbe und die Trübung des wenigstens einen vorherbestimmten Saft- und/oder Limonadenprodukts verglichen wird, und
dass die Getränkeverarbeitungsanlage basierend auf dem Ergebnis dieses Vergleichs betrieben wird.

Mit anderen Worten kann die Getränkeverarbeitungsanlage derart ausgebildet und/oder konfiguriert sein, dass sie ein oben beschriebenes Verfahren zum Verarbeiten eines Saft- und/oder Limonadenprodukts ausführen kann. Insbesondere kann das Steuerungselement derart konfiguriert und/oder ausgebildet sein, dass es ein oben beschriebenes Verfahren durchführt.

Das Steuerungselement kann beispielsweise eine speicherprogrammierbare Steuerung (SPS) und/oder ein Computer sein. Das Speicherelement kann Teil des Steuerungselements sein. Dadurch kann Farbe und Trübung einer hohen Anzahl von Ausgangsprodukten und/oder Saft-und/oder Limonadenprodukten im Speicherelement hinterlegt werden.

Die Getränkeverarbeitungsanlage kann außerdem eine Abfüllvorrichtung zum Abfüllen des Saft-und/oder Limonadenprodukts umfassen, insbesondere wobei der Sensor vor und/oder in der Abfülleinrichtung angeordnet ist.

Die Getränkeverarbeitungsanlage kann außerdem eine Mischvorrichtung umfassen, in der ein Saft- und/oder Limonadenprodukt durch Mischen von wenigstens zwei Ausgangsprodukten hergestellt wird. In diesem Fall kann der Sensor vor, in und/oder nach der Mischvorrichtung angeordnet sein.

Es können auch mehrere Sensoren, insbesondere an unterschiedlichen Positionen, in der Getränkeverarbeitungsanlage angeordnet sein. Dadurch ist eine Qualitätskontrolle an unterschiedlichen Positionen der Getränkeverarbeitungsanlage möglich.

Die Getränkeverarbeitungsanlage kann insbesondere auch einen oder mehrere Sensoren zur Bestimmung einer weiteren Eigenschaft wenigstens eines Ausgangsprodukts und/oder des Saft- und/oder Limonadenprodukts umfassen. Insbesondere kann die Getränkeverarbeitungsanlage wenigstens einen Sensor zum Bestimmen der elektrischen Leitfähigkeit, der Temperatur und/oder der Dichte des wenigstens einen Ausgangsprodukts und/oder des Saft- und/oder Limonadenprodukts umfassen.

Weitere Vorteile und Merkmale der Erfindung werden nachfolgend anhand der beispielhaften Figuren erläutert. Dabei zeigt
- Figur 1: eine Mischvorrichtung einer beispielhaften Getränkeverarbeitungsanlage;
- Figur 2: eine Füllvorrichtung einer beispielhaften Getränkeverarbeitungsanlage; und
- Figur 3: eine Illustration eines beispielhaften Verfahrens zum Verarbeiten eines Saft-und/oder Limonadenprodukts in Form eines Flussdiagramms.

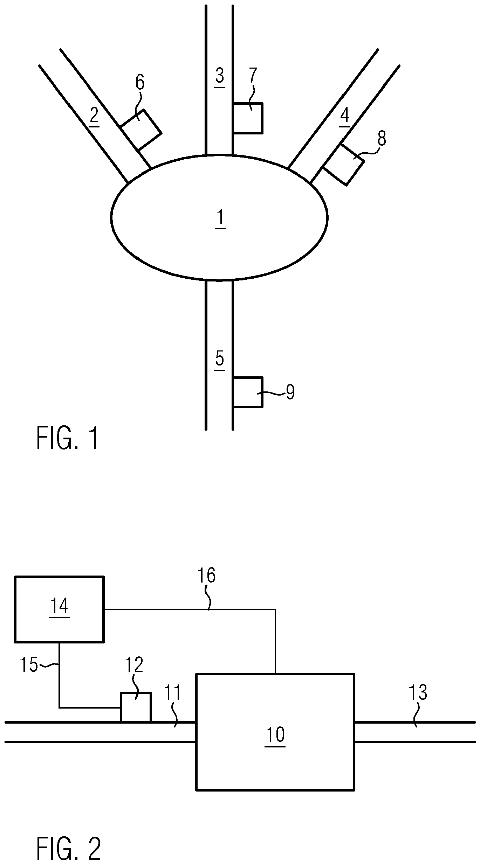

In Figur 1 ist eine beispielhafte Mischvorrichtung einer Getränkeverarbeitungsanlage schematisch dargestellt. Mit Hilfe dieser Mischvorrichtung kann ein Saft- und/oder Limonadenprodukt durch Mischen von drei Ausgangsprodukten hergestellt werden. Beispielsweise kann ein Fruchtsaftgetränk durch Mischung von drei unterschiedlichen Fruchtsäften, beispielsweise Erdbeersaft, Orangensaft und Bananensaft, hergestellt werden.

Entsprechend führen in die Mischvorrichtung 1 drei Zuleitungen 2, 3 und 4, die jeweils für ein Ausgangsprodukt des Saft- und/oder Limonadenprodukts vorgesehen sind. Zuleitung 2 kann beispielsweise für den Erdbeersaft, Zuleitung 3 für den Orangensaft und Zuleitung 4 für den Bananensaft vorgesehen sein. Die Mischvorrichtung 1 mischt diese Ausgangsprodukte oder Komponenten gemäß einem vorhergebestimmten Mischverhältnis. Das gemischte oder erhaltene Saft- und/oder Limonadenprodukt wird über die Ableitung 5 aus der Mischvorrichtung 1 ausgebracht.

In diesem Beispiel ist an jeder der Zuleitungen 2, 3 und 4 sowie an der Ableitung 5 ein Sensor 6, 7, 8 und 9 vorgesehen.

Mit den Sensoren 6, 7 und 8 können die Farbe und die Trübung des jeweiligen Ausgangsproduktes des Saft- und/oder Limonadenprodukts bestimmt werden. Der Sensor 9 ermöglicht das Bestimmen der Farbe und der Trübung des fertigen Saft- und/oder Limonadenprodukts, also der Mischung der drei Ausgangsprodukte.

Sensoren, mit denen Farbe und Trübung einer Flüssigkeit bestimmt werden können, sind grundsätzlich bekannt. So wird beispielsweise von der Firma Mettler Toledo ein entsprechender Sensor mit der Bezeichnung "InPro 8300 RAMS" vertrieben. Bislang wurden derartige Sensoren nicht beim Verarbeiten von Saft- und/oder Limonadenprodukten verwendet, da diese aufgrund ihrer Zusammensetzung häufig Schwankungen in Farbe und Trübung aufweisen. Es hat sich jedoch überraschenderweise herausgestellt, dass die bestimmte Farbe und die bestimmte Trübung auch zur Identifikation eines Saft- und/oder Limonadenprodukts bzw. eines Ausgangsprodukts eines Saft- und/oder Limonadenprodukts verwendet werden kann.

Mit Hilfe eines in Figur 1 nicht explizit dargestellten Steuerungselements der Getränkeverarbeitungsanlage kann die Getränkeverarbeitungsanlage basierend auf einem Ergebnis eines Vergleichs der mit den Sensoren 6, 7, 8 oder 9 bestimmten Farbe und Trübung mit einer gespeicherten Farbe und einer gespeicherten Trübung wenigstens eines Ausgangsprodukts eines vorherbestimmten oder gewünschten Saft- und/oder Limonadenprodukts und/oder einer gespeicherten Farbe und einer gespeicherten Trübung eines vorherbestimmten oder gewünschten Saft- und/oder Limonadenprodukts betrieben werden. Beispielsweise kann erkannt werden, ob die in die Mischvorrichtung eingebrachten Ausgangsprodukte des Saft- und/oder Limonadenprodukts den Ausgangsprodukten laut Rezept des herzustellenden Saft- und/oder Limonadenprodukts entsprechen. Gemäß obigen Beispiel sollte also über Zuleitung 2 Erdbeersaft, über Zuleitung 3 Orangensaft und über Zuleitung 4 Bananensaft in die Mischvorrichtung 1 eingebracht werden. Sollte wenigstens eines davon nicht zutreffen, kann die Getränkeverarbeitungsanlage angehalten werden und/oder kann ein Alarm oder ein Warnsignal an eine Bedienperson ausgegeben werden.

Über den Sensor 9 kann beispielsweise bestimmt werden, ob die Ausgangsprodukte im korrekten Verhältnis gemischt wurden.

Figur 2 zeigt eine beispielhafte Abfüllvorrichtung einer Getränkeverarbeitungsanlage. Über eine beispielhafte Zuleitung 11 kann ein abzufüllendes Saft- und/oder Limonadenprodukt in die Füllvorrichtung 10 eingebracht werden. Die mit dem Saft- und/oder Limonadenprodukt gefüllten Behälter, beispielsweise Flaschen, können danach über ein Transportmedium 13 in der Getränkeverarbeitungsanlage weiter transportiert werden, beispielsweise zu einem Packer.

Im Bereich der Zuleitung 11 ist ein Sensor 12 zum Bestimmen der Farbe und der Trübung des abzufüllenden Saft- und/oder Limonadenprodukts angeordnet. Über eine Datenleitung 15 ist dieser Sensor 12 mit einem Steuerungselement 14 der Getränkeverarbeitungsanlage verbunden. In dem Steuerungselement 14 können die vom Sensor 12 bestimmte Farbe und Trübung mit der gespeicherten Farbe und Trübung eines vorherbestimmten oder gewünschten Saft-und/oder Limonadenprodukts verglichen werden. Wie oben dargelegt, kann die Getränkeverarbeitungsanlage angehalten werden und/oder ein Warnsignal ausgegeben werden, wenn basierend auf dem Vergleich erkannt wird, dass in der Zuleitung 11 ein falsches Saft- und/oder Limonadenprodukt in die Abfüllvorrichtung 10 eingebracht wird.

Alternativ oder zusätzlich kann das Steuerungselement 14 mit der Abfüllvorrichtung über eine Datenleitung 16 verbunden sein. Basierend auf dem Ergebnis des Vergleichs kann dann wenigstens eine Betriebseinstellung der Abfülleinrichtung 10 verändert oder eingestellt werden. Beispielsweise kann die Abfüllmenge basierend auf dem durch den Vergleich erkannten Saft-und/oder Limonadenprodukt gewählt werden.

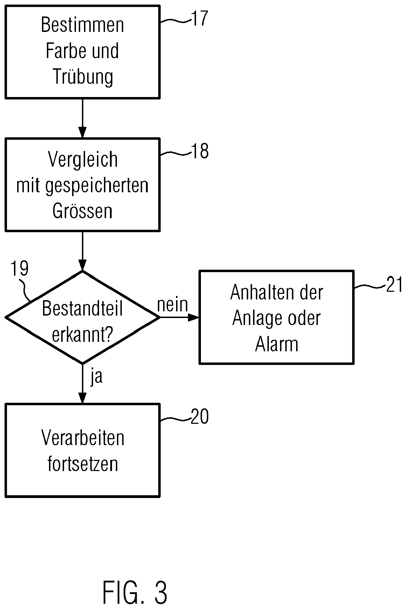

In Figur 3 wird mit Hilfe eines Flussdiagramms ein beispielhaftes Verfahren zum Verarbeiten eines Saft- und/oder Limonadenprodukts in einer Getränkeverarbeitungsanlage dargestellt.

In Schritt 17 werden zunächst die Farbe und die Trübung wenigstens eines Ausgangsprodukts eines Saft- und/oder Limonadenprodukts und/oder Farbe und Trübung eines Saft- und/oder Limonadenprodukts mittels eines Sensors der Getränkeverarbeitungsanlage bestimmt. Es können insbesondere für alle Ausgangsprodukte des Saft- und/oder Limonadenprodukts mit je einem Sensor die Farbe und die Trübung bestimmt werden.

Zusätzlich zur Farbe und Trübung kann auch noch beispielsweise die Leitfähigkeit, die Temperatur und/oder die Dichte bestimmt werden. In Schritt 18 wird die bestimmte Farbe und Trübung mit Hilfe eines Steuerungselements mit einer in einem Speicher hinterlegten Farbe und Trübung verglichen.

Im Speicher können insbesondere Farbe und Trübung aller Ausgangsprodukte eines zu verarbeitenden oder gewünschten Saft- und/oder Limonadenprodukts und/oder Farbe und Trübung des gewünschten Saft- und/oder Limonadenprodukts selbst gespeichert sein.

Wird das wenigstens eine Ausgangsprodukt beziehungsweise das Saft- und/oder Limonadenprodukt basierend auf dem Vergleich in Schritt 19 erkannt, wird in Schritt 20 das Verarbeiten des Saft- und/oder Limonadenprodukts fortgesetzt. Wird das wenigstens eine Ausgangsprodukt beziehungsweise das Saft- und/oder Limonadenprodukt nicht erkannt, kann in Schritt 21 ein Anhalten der Getränkeverarbeitungsanlage und/oder die Ausgabe eines Alarms oder Warnsignals, beispielsweise an eine Bedienperson, erfolgen.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Verfahren zum Verarbeiten, insbesondere Herstellen und/oder Abfüllen, eines Saft-und/oder Limonadenprodukts in einer Getränkeverarbeitungsanlage, wobei das Saft-und/oder Limonadenprodukt aus einem oder mehreren Ausgangsprodukten erhalten wird, umfassend die Schritte:
Bestimmen der Farbe und der Trübung wenigstens eines Ausgangsprodukts eines Saft-und/oder Limonadenprodukts und/oder der Farbe und der Trübung des erhaltenen Saft-und/oder Limonadenprodukts mittels wenigstens eines Sensors (6, 7, 8, 9) der Getränkeverarbeitungsanlage;
Vergleichen der bestimmten Farbe und Trübung mit einer gespeicherten Farbe und einer gespeicherten Trübung wenigstens eines Ausgangsprodukts eines vorherbestimmten Saft- und/oder Limonadenprodukts und/oder mit einer gespeicherten Farbe und einer gespeicherten Trübung des vorherbestimmten Saft- und/oder Limonadenprodukts; und
Betreiben der Getränkeverarbeitungsanlage basierend auf dem Ergebnis dieses Vergleichs.

2. Verfahren nach Anspruch 1, umfassend Herstellen des Saft- und/oder Limonadenprodukts wobei das Herstellen ein Mischen von wenigstens zwei Ausgangsprodukten umfasst und wobei das Bestimmen der Farbe und der Trübung vor, während und/oder nach dem Mischen durchgeführt wird.

3. Verfahren nach Anspruch 1 oder 2, umfassend Abfüllen des Saft- und/oder Limonadenprodukts wobei das Bestimmen der Farbe und der Trübung vor und/oder während des Abfüllens durchgeführt wird.

4. Verfahren nach einem der vorangegangenen Ansprüche, wobei das Vergleichen ein Erkennen des wenigstens einen Ausgangsprodukts des Saft- und/oder Limonadenprodukts umfasst.

5. Verfahren nach einem der vorangegangenen Ansprüche, wobei das Betreiben der Getränkeverarbeitungsanlage basierend auf dem Ergebnis des Vergleichs ein Anhalten, einen Betriebsmoduswechsel und/oder eine Ausgabe eines Warnsignals umfasst.

6. Verfahren nach einem der vorangegangenen Ansprüche, wobei die Schritte des Verfahrens mehrfach, insbesondere periodisch oder zu vorherbestimmten Zeitintervallen, durchgeführt werden.

7. Verfahren nach einem der vorangegangenen Ansprüche, wobei der Schritt des Vergleichens durch eine Steuerungsvorrichtung (14) der Getränkeverarbeitungsanlage durchgeführt wird.

8. Verfahren nach einem der vorangegangenen Ansprüche, wobei das Vergleichen ein Bestimmen umfasst, ob die bestimmte Farbe und Trübung mit der gespeicherten Farbe und der gespeicherten Trübung gemäß eines vorherbestimmten Kriteriums übereinstimmt.

9. Verfahren nach einem der vorangegangenen Ansprüche, wobei das Betreiben der Getränkeverarbeitungsanlage basierend auf dem Ergebnis des Vergleichs ein Ändern oder Einstellen wenigstens eines Verarbeitungsparameters der Getränkeverarbeitungsanlage basierend auf dem Ergebnis des Vergleichs umfasst.

10. Getränkeverarbeitungsanlage zum Verarbeiten, insbesondere Herstellen und/oder Abfüllen, eines Saft- und/oder Limonadenprodukts, wobei das Saft- und/oder Limonadenprodukt aus einem oder mehreren Ausgangsprodukten erhalten wird, umfassend:
wenigstens einen Sensor (6, 7, 8, 9) zum Bestimmen der Farbe und der Trübung wenigstens eines Ausgangsprodukts eines Saft- und/oder Limonadenprodukts und/oder der Farbe und der Trübung des erhaltenen Saft- und/oder Limonadenprodukts;
ein Speicherelement, in dem die Farbe und die Trübung wenigstens eines Ausgangsprodukts wenigstens eines vorherbestimmten Saft- und/oder Limonadenprodukts und/oder die Farbe und die Trübung des wenigstens einen vorherbestimmten Saft-und/oder Limonadenprodukts gespeichert ist; und
ein Steuerungselement (14), das derart konfiguriert und/oder ausgebildet ist,
dass eine durch den wenigstens einen Sensor (6, 7, 8, 9) bestimmte Farbe und Trübung mit der gespeicherten Farbe und der gespeicherten Trübung wenigstens eines Ausgangsprodukts wenigstens eines vorherbestimmten Saft- und/oder Limonadenprodukts und/oder die Farbe und die Trübung des wenigstens einen vorherbestimmten Saft- und/oder Limonadenprodukts verglichen wird; und
dass die Getränkeverarbeitungsanlage basierend auf dem Ergebnis dieses Vergleichs betrieben wird.
